# EUROPÄISCHE PATENTANMELDUNG

(11) **EP 0 920 847 A2**
(43) Veröffentlichungstag der Anmeldung: **09.06.1999**
(21) Anmeldenummer: 98122753.1
(22) Anmeldetag: 01.12.1998
(51) Int. Cl.: A61F 13/56

(54) **Verschlussband für Windeln**

(30) Priorität: 03.12.1997 DE 19753536; 12.02.1998 DE 19805576
(71) Anmelder: Koester GmbH & Co. KG, 96146 Altendorf (DE)
(72) Erfinder: Bräunig, Werner, 97514 Oberaurach 2 (DE)
(74) Vertreter: Castell, Klaus, Dr.

(57) **Zusammenfassung**

Das Windelverschlußband hat einen ersten Befestigungsbereich, um das Band an einem hinteren Windelohr zu befestigen. Auf dem Verschlußband ist lösbar eine Klebstoffschicht aufgebracht, indem zwischen Klebstoffschicht und Trägermaterial eine Silikonschicht vorgesehen ist. Beim Umlegen des Verschlußbandes um das hintere Windelohr wird die Klebstoffschicht auf die Innenseite des hinteren Windelohres aufgeklebt. Während der Windeltragzeit wird durch diese Klebstoffschicht das Randende des vorderen Windelohrs am hinteren Windelohr gehalten.

Beim Abziehen des Verschlußbandes von der Innenseite des hinteren Windelohres löst sich die Klebstoffsicht von der Silikonschicht und verbleibt an der Innenseite des hinteren Windelohrs.

## Beschreibung

Die Erfindung betrifft ein Verschlußband mit einem ersten Befestigungsbereich zum Befestigen des Bandes an einem ersten Bereich, insbesondere einer Windel.

Insbesondere zum Verschluß von Windeln sind verschiedenartigste Verschlußbänder bekannt. Die bekannten Ausführungsformen haben jedoch den Nachteil, daß sie einerseits sehr aufwendig in der Fertigung sind und andererseits häufig nicht vermeidbar ist, daß harte Bereiche am Windelverschlußband mit der Haut des Windelträgers in Berührung kommen. Insbesondere beim Zurückklappen des vorderen Windelohres während der Tragdauer entsteht leicht ein Kontakt zwischen dem Windelverschlußband und der Haut des Windelträgers. Dieser Kontakt führt insbesondere bei Kleinkindern häufig zu Hautirritationen bis hin zu Ausschlägen.

Aufgabe der Erfindung ist es daher, ein einfaches Verschlußband herzustellen, das bei der Verwendung als Windelverschlußband einen Kontakt zwischen dem Windelverschlußband und der Haut des Windelträgers vermeidet.

Diese Aufgabe wird dadurch gelöst, daß auf dem Verschlußband lösbar eine Klebstoffschicht aufgebracht ist.

Die lösbar am Verschlußband angebrachte Klebstoffschicht kann mit dem Windelverschlußband auf einen Bereich der Windel aufgelegt werden, so daß die Klebstoffschicht in diesem Bereich anklebt. Anschließend kann das Verschlußband in diesem Bereich von der Klebstoffschicht abgelöst werden, so daß in diesem Bereich eine voll funktionsfähige Klebstoffschicht verbleibt.

Beim Verwenden des Verschlußbandes als Windelverschlußband kann der erste Windelbereich, an dem das Verschlußband befestigt ist, an der Außenseite des hinteren Windelohres liegen. Das Windelverschlußband kann dann mit der lösbar aufgebrachten Klebstoffschicht auf die Innenseite des hinteren Windelohres aufgelegt werden, so daß die Klebstoffschicht das Windelverschlußband im umgeklappten Zustand hält. Vor der Verwendung der Windel wird das Windelverschlußband von der Klebstoffschicht abgezogen, so daß es nur noch über den ersten Befestigungsbereich mit der Außenseite des hinteren Windelohres in Verbindung steht. Dies ermöglicht es, den Klebebereich zur Verbindung der Innenseite des hinteren Windelohrs mit der Außenseite des vorderen Windelohres zu verwenden. Dadurch wird gewährleistet, daß sich zwischen dem Windelverschlußband und der Haut immer das vordere weiche Windelohr befindet und diese schützt. Außerdem kann die auf die Innenseite des hinteren Windelohres transferierte Klebstoffschicht dazu verwendet werden, die gebrauchte Windel nach ihrer Benutzung in einem zusammengerollten Zustand zu halten.

Eine vorteilhafte Ausführungsform des Verschlußbandes sieht vor, daß das Verschlußband einen zweiten Befestigungsbereich zum Befestigen des Bandes an einem zweiten Bereich, insbesondere einer Windel aufweist, und die Klebstoffschicht nur im Transportzustand einer Verschlußbandrolle zwischen erstem und zweitem Befestigungsbereich angeordnet ist. Beim Gebrauch der Windel ist zwischen erstem und zweitem Befestigungsbereich keine Klebstoffschicht vorhanden. Die Klebstoffschicht befindet sich beim Anwenden der Windel an der Innenseite des hinteren Windelohres. Die erfindungsgemäße Klebstoffschicht kann somit in bekannte Windelverschlußbänder integriert werden.

Ein im praktischen Einsatz bewährtes Windelverschlußband weist ein Trägermaterial auf, auf dem als erster Befestigungsbereich eine Klebstoffschicht aufgebracht ist. Als zweiter Befestigungsbereich dient vorzugsweise ein mechanisches Verschlußteil, das ebenfalls aufdem Trägermaterial angeordnet ist. Während das mechanische Verschlußteil die Hauptkräfte beim Verschließen der Windel abfängt, verhindert die transferierte Klebstoffschicht ein Umklappen des vorderen Windelohres und vermeidet somit einen Kontakt zwischen dem Windelverschlußband und der Haut des Windelträgers.

Ein einfaches, leichtes Ablösen der Klebstoffschicht wird dadurch erzielt, daß das Band ein Trägermaterial aufweist, auf dem eine dehäsive Fläche zum Aufbringen der Klebstoffschicht vorgesehen ist. Die dehäsive Fläche ermöglicht zwar das Aufbringen der Klebstoffschicht. Ein permanentes Festkleben der Klebstoffschicht am Trägermaterial wird durch die dehäsive Fläche jedoch vermieden.

Ein bevorzugtes Ausführungsbeispiel sieht vor, daß die dehäsive Fläche eine Silikonfläche ist. Eine Silikonschicht ist einfach aufzubringen und verhindert hinreichend ein permanentes Verkleben der Klebstoffschicht mit dem Trägermaterial.

Vorzugsweise ist die Klebstoffschicht so ausgebildet, daß sie an einem Windelmaterial reversibel befestigbar ist. Um die Windel nach Gebrauch wieder zu öffnen, muß die durch die Klebstoffschicht herbeigeführte Verbindung gelöst werden. Je nach Ausbildung der Klebstoffschicht verbleibt die Schicht entweder an der Innenseite des hinteren Windelohres oder an der Außenseite des vorderen Windelohrs. Eine dieser beiden Verbindungen muß jedoch lösbar sein, damit die Windel problemlos geöffnet werden kann und anschließend die Klebstoffschicht zum Halten der eingerollten gebrauchten Windel weiterverwendet werden kann.

Eine alternative Ausgestaltung sieht vor, daß das Band ein Trägermaterial aufweist und die Klebstoffschicht mittels eines mechanischen Verschlußteils am Trägermaterial angebracht ist. Auch ein mechanisches Verschlußteil beispielsweise aus Ösen und Haken erlaubt es, eine Klebstoffschicht lösbar am Band anzubringen. Das mechanische Verschlußteil ist dabei zunächst zwischen Klebstoffschicht und Trägermaterial angeordnet. Wenn die Klebstoffschicht auf die Windel und insbesondere auf die Innenseite des hinteren Windelohres aufgelegt wird, verbindet sich die Klebstoffschicht derart mit der Windel, daß beim Abziehen des Trägermaterials sich das mechanische Verschlußteil öffnet und ein Teil des mechanischen Verschlußteils an der Klebstoffschicht verbleibt und das andere Teil des mechanischen Verschlußteils am Trägermaterial. Das an der Windel verbleibende mechanische Verschlußteil kann dann anschließend mit einem Gegenstück beispielsweise an der Außenseite des vorderen Windelohres zusammenwirken.

Beispielsweise kann das Trägermaterial aus einem stoffähnlichen (clothlike) Material bestehen. Die Klebstoffschicht kann dann auf der dem Trägermaterial zugewandten Seite ein Hakenmaterial aufweisen mit dem die Klebstoffschicht mit dem Trägermaterial in Verbindung steht. Beim Auflegen der Klebstoffschicht auf der Innenseite des hinteren Windelohres verbleibt dann die Klebstoffschicht mitsamt dem Hakenmaterial am hinteren Windelohr. Das so ausgerüstete hintere Windelohr kann anschließend mit einem stoffähnlichen Material am vorderen Windelohr zusammenwirken. Als stoffähnliches Material ist vorzugsweise ein Schlaufenmaterial vorzusehen.

Die schon oben beschriebene bevorzugte Verwendung des Verschlußbandes dient dem Verschließen einer Windel. Dazu wird das Verschlußband an einem äußeren hinteren Teil einer Windels mittels des ersten Befestigungsbereichs befestigt. Anschließend wird auf den inneren hinteren Teil der Windel die Klebstoffschicht aufgelegt und beim Anlegen der Windel wird das Verschlußband von dem inneren hinteren Teil der Windel abgezogen, wobei die Klebstoffschicht am inneren hinteren Teil der Windel verbleibt.

Bei Verwendung einer dehäsiven Fläche oder einer Silikonschicht wird mittels des erfindungsgemäßen Verfahrens eine klebende Fläche an der inneren Seite des hinteren Windelohres bereitgestellt. Sofern ein mechanisches Verschlußteil zum lösbaren Anbringen der Klebstoffschicht verwendet wird, kann an der Innenseite des hinteren Windelohres, das eine Teil - vorzugsweise das Hakenmaterial - eines mechanischen Verschlusses angebracht werden. Das Anbringen des Verbindungselementes zwischen hinterem und vorderem Windelohr auf der Anlagefläche zwischen den Ohren hat den Vorteil, daß das vordere Windelohr nicht umknickt und somit eine Berührung zwischen dem Windelverschlußband und der Haut des Windelträgers ausgeschlossen ist.

Zwei Ausführungsbeispiele des erfindungsgemäßen Verschlußbandes sind in der Zeichnung dargestellt und werden im folgenden ausführlich erläutert. Es zeigt,
- Figur 1: schematisch, eine geschlossene bekannte Windel,
- Figur 2: schematisch, ein am hinteren Windelohr befestigtes Verschlußband,
- Figur 3: schematisch, ein auf die Innenseite des hinteren Windelohres aufgelegtes Verschlußband,
- Figur 4: schematisch, ein wieder aufgeklapptes Verschlußband mit dem ein vorderes Windelohr am hinteren Windelohr befestigt ist und
- Figur 5: eine alternative Ausführungsform des Windelverschlußbandes nach Figur 2.

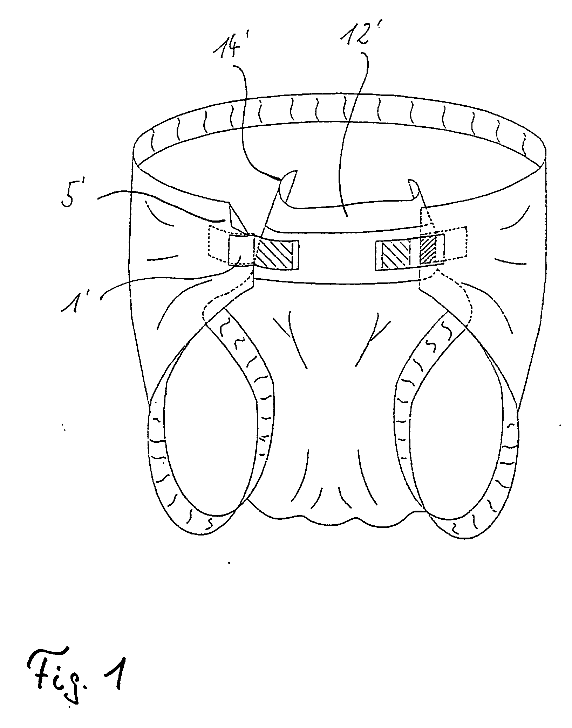

Figur 1 zeigt das bei herkömmlichen Windelverschlußbändern 1' entstehende Problem, daß das vordere Windelohr 12' gut am hinteren Windelohr 5' befestigt ist, der Randbereich 14 des vorderen Windelohres 12' aber leicht beim Tragen der Windel nach innen umklappt, wobei harte Bereiche des Windelverschlußbandes 1' freigelegt werden.

Das in Figur 2 gezeigte Windelverschlußband 1 besteht aus einem Trägermaterial 2, auf dessen einer Seite als Befestigungsbereich 3 eine Klebstoffschicht aufgebracht ist. Diese Klebstoffschicht dient dazu, das Windelverschlußband 1 an der Außenseite 4 eines hinteren Windelohres 5 zu befestigen. Am gegenüberliegenden Ende des Trägermaterials 2 befindet sich zunächst eine Grifflasche 6 an die sich zum Befestigungsbereich 3 hin, eine Klebstoffschicht 7 mit einem darauf aufgebrachten Hakenmaterial 8 befindet. Daran in Richtung zum Befestigungsbereich 3 anschließend ist auf das Trägermaterial 2 eine Silikonschicht 9 aufgebracht und auf diese Silikonschicht 9 ist eine weitere Klebstoffschicht 10 aufgelegt.

Die Figur 3 zeigt, das im Transportzustand um das hintere Windelohr 5 herumgelegte Verschlußband 1. Dabei liegt die Klebstoffschicht 10 auf der Innenseite 11 des hinteren Windelohres 5 und auch das Hakenmaterial liegt an der Innenseite 11 des hinteren Windelohres 5 an. Das umgefaltete Verschlußband klebt mit der Klebstoffsicht so fest, daß das umgeklappte Stück während der Behandlung in der Windelmaschine nicht aufklappt.

Um das hintere Windelohr 5 mittels des Windelverschlußbandes 1 an einem vorderen Windelohr 12 zu befestigen, wird zunächst das Windelverschlußband 1 wieder aufgeklappt, wobei die Klebstoffschicht 10 jedoch an der Innenseite 11 des hinteren Windelohres 5 verbleibt. Die Silikonschicht 9 ist so ausgebildet, daß sie die Klebstoffschicht 10 weniger fest an das Trägermaterial 2 bindet, als das Material der Innenseite 11 des hinteren Windelohres 5.

Das wieder aufgeklappte Windelverschlußband 1 kann somit mit dem Hakenmaterial 8 an einem Schlaufenmaterial 13 an der Vorderseite des vorderen Windelohres 12 befestigt werden. Der Randbereich 14 des vorderen Windelohres 12 wird mit der Klebstoffschicht 10 an der Innenseite 11 des hinteren Windelohres 5 gehalten.

In der Praxis ist der Befestigungsbereich 3 etwa 22 mm breit und mit einem permanent klebenden Kleber versehen. Daran schließt sich ein etwa 2 mm breiter Faltbereich 15 an, der das Umklappen des Trägermaterials 2 erleichtert. Im Anschluß ist ein 17 mm breiter silikonisierter Bereich 9 vorgesehen, auf dem der Transferkleber 10 angebracht ist. Dieser Kleber ist so ausgebildet, daß er sich leicht von der Silikonschicht ablöst und eine lösbare Verbindung zwischen der Innenseite 11 des hinteren Windelohres 5 und der Vorderseite des vorderen Windelohrs 12 bildet. Anstelle des Zusammenwirkens der Haken 8 und der Schlaufen 13 kann an dieser Stelle auch eine Klebeverbindung vorgesehen sein.

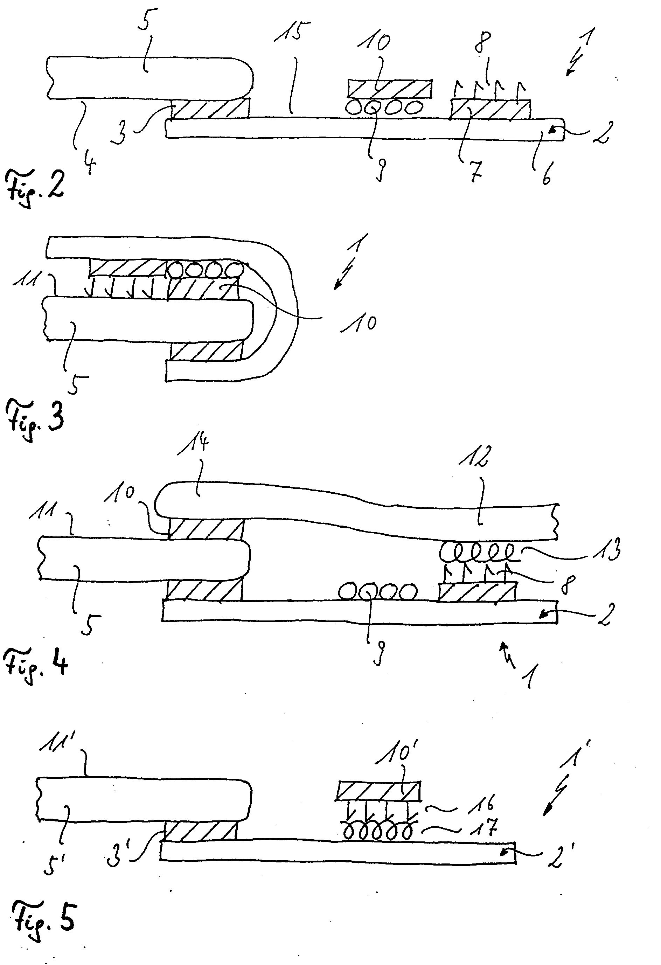

Eine alternative Ausführungsform eines erfindungsgemäßen Verschlußbandes zeigt Figur 5. Das Windelverschlußband nach Figur 5 hat ebenfalls einen Befestigungsbereich 3'. Anstelle der Silikonschicht 9 am Windelverschlußband 1 ist bei dieser Ausführungsform eine mechanische Verbindung aus Haken 16 und Schlaufenmaterial 17 als lösbare Verbindung zwischen der Klebstoffschicht 10' und dem Trägermaterial 2' vorgesehen. Auch dieses Windelverschlußband 1' ist um das hintere Windelohr 5' herumlegbar, so daß die Klebstoffschicht 10' an der Innenseite 11' des hinteren Windelohres 5' zu liegen kommt. Beim Zurückklappen des Verschlußbandes 1' verbleibt die Klebstoffschicht 1' mit dem Hakenmaterial 16 an der Innenseite 11' des hinteren Windelohres 5' und ermöglicht eine Befestigung der Innenseite 11' des hinteren Windelohres 5' an einem auf der Vorderseite eines vorderen Windelohres angebrachten Schlaufenmaterial (nicht gezeigt).

Die dem Befestigungsbereich 3' gegenüberliegende Seite des Trägermaterials 2' kann entweder als Grifflasche dienen oder es kann auf diesem Ende wie bei dem Ausführungsbeispiel nach den Figuren 2 bis 4 ein weiteres mechanisches Verschlußteil angeordnet werden.

Der erfindungsgemäße Aufbau des Verschlußbandes ermöglicht somit den Randbereich 14 eines vorderen Windelohrs 12 an der Innenseite 11 eines hinteren Windelohres 5 zu befestigen. Diese Befestigung kann ausschließlich ein Umklappen des Endes 14 vermeiden, wenn eine weitere Befestigungsmöglichkeit zwischen vorderem und hinterem Windelohr vorgesehen ist, oder als ausschließliche Befestigung zwischen vorderem und hinterem Windelohr dienen.

Darüberhinaus können die Klebstoffschicht 10 bzw. 10' und die Oberflächen der Innenseite des hinteren Windelohrs und der Außenseite des vorderen Windelohrs so aufeinander abgestimmt werden, daß bei einem Lösen der Verbindung zwischen den Windelohren die Klebstoffschicht 10 bzw. 10' an der Innenseite des hinteren Windelohrs verbleibt und ein Verschließen der Windel im eingerollten, gebrauchten Zustand ermöglicht.

## Patentansprüche

1. Verschlußband (1, 1') mit einem ersten Befestigungsbereich (3, 3') zum Befestigen des Bandes (1, 1') an einem ersten Bereich, insbesondere einer Windel, ***dadurch gekennzeichnet, daß*** auf dem Verschlußband (1, 1') lösbar eine Klebstoffschicht (10, 10') aufgebracht ist.

2. Verschlußband nach Anspruch 1, ***dadurch gekennzeichnet, daß*** das Verschlußband (1) einen zweiten Befestigungsbereich (8) zum Befestigen des Bandes (1) an einem zweiten Bereich, insbesondere einer Windel aufweist und die Klebstoffschicht **nur im Transportzustand einer Verschlußbandrolle** (10) zwischen erstem (3) und zweitem (8) Befestigungsbereich angeordnet ist.

3. Verschlußband nach einem der vorhergehenden Ansprüche, ***dadurch gekennzeichnet, daß*** das Verschlußband ein Trägermaterial (2) aufweist, auf dem als erster Befestigungsbereich (3) eine Klebstoffschicht aufgebracht ist.

4. Verschlußband nach einem der vorhergehenden Ansprüche, ***dadurch gekennzeichnet, daß*** das Verschlußband ein Trägermaterial (2) aufweist, an dem als zweiter Befestigungsbereich ein mechanisches Verschlußteil (8) angeordnet ist.

5. Verschlußband nach einem der vorhergehenden Ansprüche, ***dadurch gekennzeichnet, daß*** das Verschlußband ein Trägermaterial (2) aufweist, auf dem eine dehäsive Fläche (9) zum Aufbringen der Klebstoffschicht (10) vorgesehen ist.

6. Verschlußband nach Anspruch 5, ***dadurch gekennzeichnet, daß*** die dehäsive Fläche eine Silikonschicht (9) ist.

7. Verschlußband nach einem der vorhergehenden Ansprüche, ***dadurch gekennzeichnet, daß*** die Klebstoffschicht (10) so ausgebildet ist, daß sie an einem Windelmaterial reversibel befestigbar ist.

8. Verschlußband nach einem der Ansprüche 1 bis 4, ***dadurch gekennzeichnet, daß*** das Band ein Trägermaterial (2') aufweist und die Klebstoffschicht (10') mittels eines mechanischen Verschlußteils (16, 17) am Trägermaterial (2') angebracht ist.

9. Verschlußband nach einem der vorhergehenden Ansprüche*, **dadurch gekennzeichnet, daß*** das Trägermaterial (2') eine textile Struktur aufweist.

10. Verfahren zur Verwendung eines Verschlußbandes (1, 1') nach einem der Ansprüche 1 bis 9, ***dadurch gekennzeichnet, daß*** das Verschlußband (1, 1') an einem äußeren hinteren Teil (5, 5') einer Windel mittels des ersten Befestigungsbereichs (3, 3') befestigt wird, auf den inneren hinteren Teil der Windel (5, 5') die Klebstoffschicht (10, 10') aufgelegt wird und beim Anlegen der Windel das Verschlußband (1, 1') von dem inneren hinteren Teil der Windel abgezogen wird, wobei die Klebstoffschicht (10, 10') am inneren hinteren Teil (11') der Windel verbleibt.
